# EUROPEAN PATENT APPLICATION

(11) **EP 1 767 513 A1**
(43) Date of publication of application: **28.03.2007**
(21) Application number: 05755786.0
(22) Date of filing: 30.06.2005
(51) Int. Cl.: C07C 27/00, C07C 29/56, C07C 35/37, C07C 45/29, C07C 49/453, C07B 61/00

(54) **METHOD FOR PRODUCING 2-ADAMANTANOL AND 2-ADAMANTANONE**

(30) Priority: 09.07.2004 JP 2004203878
(71) Applicant: IDEMITSU KOSAN CO., LTD., Tokyo 100-8321 (JP)
(72) Inventor: KOJIMA, Akio, 2990193 (JP); YAMANE, Hideki, 2990193 (JP); OKAMOTO, Kenji, 2990193 (JP)
(74) Representative: Vossius & Partner
(86) International application number: PCT/JP2005/012052
(87) International publication number: WO 2006/006413

(57) **Abstract**

The present invention is a process for producing 2-adamantanol and 2-adamantanone from 1-adamantanol, by using as a catalyst a substance comprising at least one kind of acid catalyst selected from Lewis acid(s) and solid acid(s) that coexist with at least one kind selected from the group consisting of carboxylic acids, sulfonic acids, and phosphoric acids, and provides a process suitable for mass production of 2-adamantanol and 2-adamantanone selectively with high efficiency without using sulfuric acid as a catalyst, thereby enabling laborsaving in waste acid treatment step and drastic reduction of the reaction time.

## Description

### TECHNICAL FIELD

The present invention relates to a process for producing 2-adamantanol and 2-adamantanone, which are important intermediates among adamantane derivatives as raw materials for various pharmaceutical and agricultural chemicals and industrial products, by oxidizing 1-adamantanol in a short time without using sulfuric acid with due consideration to the environment.

### BACKGROUND ART

Adamantane is known as a basket-type compound with a high degree of symmetry having the same structure as the structural unit of diamond. As a chemical substance, it has characteristics such as (1) low molecular distortion energy and high thermal stability, (2) high solubility in lipid because of its high carbon density, (3) low level of odor in spite of its sublimation properties and the like, and hence it has attracted attention as a raw material of drugs for Parkinson's disease and influenza in the area of pharmaceutical products since 1980s. In recent years, however, the characteristics of the adamantane derivatives such as thermal stability and transparency have attracted attention in the areas of optical materials such as photo resist for manufacturing semiconductors, magnetic recording media, optical fibers, optical lenses, substrate for optical disks and the like, functional materials such as thermoresistant plastics, paints, adhesives and the like, cosmetics, lubricating oils and the like, and its applications have been growing. Also, in the pharmaceutical area, demands as a raw material for anticancer drugs, brain function ameliorators, drugs for neurological disorders, antiviral agents and the like has been growing.

Technology to convert hydrocarbon compounds to alcohols or ketones by oxidation is an industrially very important technology from the viewpoint of effective utilization of carbon resources. As a technology for producing 2-adamantanone which is an important intermediate as a raw material for various pharmaceutical and agricultural chemicals and industrial products, a process for producing it in concentrated sulfuric acid is publicly known. For example, Schlatmann reported that 2-adamantanone is obtained with 72% yield by heating 1-adamantanol in concentrated sulfuric acid at 30°C for 12 hours (for example, refer to Non-patent Document 1). It is also known that adamantanone is obtained with 47 to 48% yield by oxidation of adamantane with concentrated sulfuric acid followed by purification with steam distillation (for example, refer to Non-patent Document 2). Further, as an improved method thereof, a method is proposed, wherein the reaction is performed by raising temperature in two or three stages (for example, refer to Patent Documents 1 and 2).
However, although the yield of 2-adamantanone increases up to 90%, these methods have a lot of challenges when mass production is aimed at because a massive amount of concentrated sulfuric acid is used, and therefore (a) an enormous amount of waste acid is generated, (b) expensive corrosion-resistant equipment material is needed, and (c) long reaction time (12 to 24 hours) is required.
As for the current situation, there is no technology that uses no sulfuric acid as an oxidizing agent, and therefore, it is the situation where introduction of a production technology for 2-adamantanone without using sulfuric acid is long awaited.

[Non-patent Document 1] Tetrahedron: 24, 5361 (1968)
[Non-patent Document 2] Organic Syntheses 53, 8 (1973)
[Patent Document 1] Japanese Patent Laid-open Publication No. H11-189564
[Patent Document 2] Japanese Patent Laid-open Publication No. 2003-267906

### DISCLOSURE OF THE INVENTION

An object of the present invention is to provide a process for producing 2-adamantanol and 2-adamantanone selectively and efficiently in an unconventionally short time with due consideration to the environment by using an acid catalyst on 1-adamantanol.
To resolve the aforementioned conventional technological problems and to produce 2-adamantanol and 2-adamantanone selectively and efficiently in a short time with due consideration to the environment, the inventors of the present invention pursued intensive study, and have found that the above mentioned problems can be solved by reacting 1-adamantanol using a catalyst such as a Lewis acid or a solid acid catalyst with which coexist at least one kind selected from the group consisting of carboxylic acids, sulfonic acids, and phosphoric acids, and thus have achieved the above object. The present invention has thus been accomplished based on the foregoing findings.
Accordingly, the present invention is to provide:
(1) a process for producing 2-adamantanol and 2-adamantanone from 1-adamantanol, wherein a substance comprising at least one kind of acid catalyst selected from Lewis acid(s) and solid acid(s) that coexist with at least one kind selected from the group consisting of carboxylic acids, sulfonic acids, and phosphoric acids is used as a catalyst,
(2) the process for producing 2-adamantanol and 2-adamantanone of above described (1), wherein the solid acid is at least one kind selected from zeolite, zirconia, silica-alumina, alumina, heteropoly acid, and cation exchange resin,
(3) the process for producing 2-adamantanol and 2-adamantanone of above described (1) or (2), wherein the amount of at least one kind selected from the group consisting of carboxylic acids, sulfonic acids, and phosphoric acids coexisting with the acid catalyst is 150 molar ratio or less against 1-adamantanol,
(4) the process for producing 2-adamantanol and 2-adamantanone of any of above described (1) to (3), wherein the reaction temperature is in a range from 30 to 250°C, and
(5) the process for producing 2-adamantanol and 2-adamantanone of any of above described (1) to (4), wherein the acid coexisting with the acid catalyst is at least one kind selected from halogenated carboxylic acid, halogenated sulfonic acid, and alkyl sulfonic acid.

### BEST MODE FOR CARRYING OUT THE INVENTION

The present invention is explained in detail as follows.
With respect to the process for producing 2-adamantanol and 2-adamantannone of the present invention, in the process for producing 2-adamantanol and 2-adamantanone from 1-adamantanol, a substance comprising at least one kind selected from the group consisting of carboxylic acids, sulfonic acids, and phosphoric acids coexisting with Lewis acid(s) or solid acid(s) is used as a catalyst.

As the acid catalyst in the present invention, at lease one kind selected from Lewis acid(s) and solid acid(s) is used.
As the Lewis acid, for example, aluminum chloride, ferric chloride, tin tetrachloride, titanium tetrachloride, boron trifluoride, boron trifluoride complex, boron tribromide, aluminum bromide, gallium chloride, gallium bromide and the like can be listed.

As the solid acid, at least one kind selected from zeolite, zirconia, silica-alumina, alumina, heteropoly acid, and cation exchange resin is used.
As the zeolite, A type, L type, X type, Y type, ZSM-5 and the like can be listed, and H-form ultrastable Y type zeolite (HUSY) which is acid treated NH₄-form Y type zeolite using sulfuric acid is preferably used.
Also, zirconia, silica-alumina, alumina and the like attached further with hydrochloric acid, sulfuric acid, phosphoric acid, boron trifluoride and the like, for example sulfated zirconia, can be used.
The heteropoly acid is a poly acid having a polynuclear structure wherein two or more kinds of oxo acids are condensed, and phosphorous, silicon, arsenic and germanium are used much as a heteroatom, and molybdenum, tungsten, niobium, vanadium and the like are used much as a polyatom. Specifically, silicotungstic acid, silicomolybdic acid and the like can be listed.
Also, the cation exchange resin is an insoluble and porous synthetic resin that has an exchangeable ion, for example, a polymer acid wherein an acidic group such as phenolic hydroxyl group, carboxyl group, sulfonic group and the like is bonded to parent synthetic resin such as polystyrene crosslinked with divinylbenzene and the like.
Among the above described acid catalysts, aforementioned solid acid is used preferably, and especially zeolite and heteropoly acid are used more preferably.

Also, the amount of at least one kind to be added selected from the group consisting of carboxylic acids, sulfonic acids, and phosphoric acids coexisting with the acid catalyst is, for example, generally 150 mol or less, preferably 1 to 100 mol with respect to 1 mol of 1-adamantanol. By setting the amount of at least one kind to be added selected from the group consisting of carboxylic acids, sulfonic acids, and phosphoric acids in the range as described above, the oxidation reaction rate can be increased.
As the aforementioned carboxylic acids, monocarboxylic acids such as formic acid, acetic acid, propionic acid, butyric acid and the like, dicarboxylic acids such as oxalic acid, malonic acid, succinic acid and the like, aromatic carboxylic acids such as benzoic acid, phthalic acid and the like, halogenated carboxylic acids such as monofluoroacetic acid, difluoroacetic acid, trifluoroacetic acid, monochloroacetic acid, dichloroacetic acid, trichloroacetic acid and the like can be listed.
Also, as the sulfonic acids, alkyl sulfonic acids such as methanesulfonic acid, ethanesulfonic acid and the like, aromatic sulfonic acids such as benzenesulfonic acid, toluenesulfonic acid and the like, halogenated sulfonic acids such as trifluoromethenesulfonic acid and the like can be listed.
Also, as the phosphoric acids, orthophosphoric acid, methaphosphoric acids, pyrophosphoric acids, polyphosphoric acids and the like can be listed.
Among them, halogenated carboxylic acid, halogenated sulfonic acid, and alkyl sulfonic acid are preferable, and especially halogenated carboxylic acid and halogenated sulfonic acid are more preferable.
These carboxylic acids, sulfonic acids, and phosphoric acids can be used singly or in a combination of two or more kinds.

Also, the reaction temperature is generally preferably in a range from 30 to 250°C, and more preferably in a range from 50 to 250°C. By performing the reaction in the range of temperature as described above, heavy fraction due to side reaction is reduced and decease in the selectivity to 2-adamantanone or 2-adamantanol can be prevented.
The reaction time depends on the reaction temperature, the amount of acid catalyst to be used, the kind or the amount of coexisting carboxylic acids, sulfonic acids and phosphoric acids, and the amount of 1-adamantanol and the like, and cannot be specified in general, but in the batch-wise reaction, it is usually 0.5 to 20 hours, preferably 1 to 10 hours.
Also, in the fixed-bed flow reaction, weight hourly space velocity (WHSV) with reference to 1-adamantanol is in the range from 0.001 to 50 h⁻¹, preferably 0.005 to 20 h⁻¹, where the oxidation reaction is performed preferably.

As the oxidation reaction used in the present invention, generally a method is used to proceed the reaction by using carboxylic acids, sulfonic acids, and phosphoric acids to be coexisting with the acid catalyst also as a solvent, in which a given amount of 1-adamantanol is dissolved in the presence of the acid catalyst, and then raising the temperature.
As the solvent, it is preferable to use the aforementioned carboxylic acids, sulfonic acids, and phosphoric acids which are used as additives, but it is also possible to use a solvent as necessary for the purpose of proceeding the reaction moderately. However, in that case, it is necessary to select the solvent that dissolves 1-adamantanol and is stable against the acid catalyst.
For such solvent, for example, halogenated hydrocarbon compounds such as ethylene dichloride and the like, benzene derivatives that have an inert substituent group such as nitrobenzene, chlorobenzene and the like can be listed.

Also, there is no particular limitation to the reaction apparatus as long as it allows adequate stirring and heating, and it is not necessary to use expensive corrosion resistant material since sulfuric acid is not used as an oxidizing agent.
After the oxidation reaction, 2-adamantanone and 2-adamantanol can be separated from the reaction system according to conventional procedures.

### EXAMPLES

In the following, Examples are described to further illustrate the present invention; however it is to be understood that the present invention is not intended to be limited to these Examples.
Quantitative analysis of raw materials and the products was performed by the gas chromatography based on the internal standard method.

### Example 1

0.4 g of 1-adamantanol and 20 g of monochloroacetic acid were prepared in a 100 mL three-neck flask. The resultant mixture was stirred and its temperature was raised to 150°C. After the temperature reached 150°C, 5 g of silicotungstic acid which had been dried at 300°C for 2 hours was added to the mixture, and the reaction was performed for 2 hours. After the reaction was completed, the reaction solution was filtrated, and NaOH aqueous solution was added with cooling until pH of the resultant solution became 9. Then, the solution was extracted with 20 mL of toluene. The extract was analyzed by gas chromatography. The results are shown in Table 1.

### Example 2

The reaction, after-treatment, and analysis were performed exactly in the same way as in Example 1 except that 2 g of H-form ultrastable Y type zeolite [(HUSY) with a silica-alumina ratio, SiO₂/Al₂O₃ = 10] which had been dried at 300°C for 3 hours was used instead of 5 g of silicotungstic acid. The results are shown in Table 1.

### Example 3

The reaction, after-treatment, and analysis were performed exactly in the same way as in Example 1 except that trichloroacetic acid was used instead of monochloroacetic acid. The results are shown in Table 1.

### Example 4

The reaction, after-treatment, and analysis were performed exactly in the same way as in Example 1 except that trifluoroacetic acid was used instead of monochloroacetic acid. The results are shown in Table 1.

### Example 5

The reaction, after-treatment, and analysis were performed exactly in the same way as in Example 1 except that acetic acid was used instead of monochloroacetic acid. The results are shown in Table 1.

### Example 6

The reaction, after-treatment, and analysis were performed exactly in the same way as in Example 1 except that methanesulfonic acid was used instead of monochloroacetic acid. The results are shown in Table 1.

### Comparative example 1

The reaction, after-treatment, and analysis were performed exactly in the same way as in Example 1 except that 20 g of n-tetradecane was added as a solvent without adding monochloroacetic acid, but no reaction proceeded.

### Comparative example 2

The reaction, after-treatment, and analysis were performed exactly in the same way as in Example 2 except that 20 g of n-tetradecane was added as a solvent without adding monochloroacetic acid, but no reaction proceeded.

### Comparative example 3

The reaction, after-treatment, and analysis were performed exactly in the same way as in Example 1 except that silicotungstic acid was not added, but no reaction proceeded.

### Reference example 1

The reaction, after-treatment, and analysis were performed exactly in the same way as in Example 1 except that 20 g of sulfuric acid was used as an acid catalyst and additive. The results are shown in Table 1.

**Table 1**

| | Additives | Acid catalyst | Yield of 2-adamantanol (mol %) | Yield of 2-adamantanon (mol %) | Reaction time (h) |
|---|---|---|---|---|---|
| Example 1 | Monochloroacetic acid | Silicotungstic acid | 11 | 24 | 2 |
| Example 2 | Monochloroacetic acid | HUSY (Y type zeolite) | 22 | 15 | 2 |
| Example 3 | Monochloroacetic acid | Silicotungstic acid | 12 | 24 | 2 |
| Example 4 | Acetic acid | Silicotungstic acid | 4 | 13 | 2 |
| Example 5 | Trifluoroaceitc acid | Silicotungstic acid | 11 | 23 | 2 |
| Example 6 | Methanesulfonic acid | Silicotungstic acid | 12 | 22 | 2 |
| Comparative example 1 | None | Silicotungstic acid | 0 | 0 | 2 |
| Comparative example 2 | None | HUSY (Y type zeolite) | 0 | 0 | 2 |
| Comparative example 3 | Monochloroacetic acid | None | 0 | 0 | 2 |
| Reference example 1 | 98% Sulfuric acid | | 0 | 2 | 2 |

### INDUSTRIAL APPLICABILITY

The present invention is a process for producing 2-adamantanone and 2-adamantanol in a short time with high efficiency, which is useful in the areas of pharmaceutical and agricultural chemicals, semiconductors, magnetic recording media, optical materials, thermoresistant plastics, functional materials such as paint and adhesive and the like, cosmetics, lubricating oils and the like.

## Claims

1. A process for producing 2-adamantanol and 2-adamantanone from 1-adamantanol, wherein a substance comprising at least one kind of acid catalyst selected from Lewis acid(s) and solid acid(s) that coexist with at least one kind selected from the group consisting of carboxylic acids, sulfonic acids, and phosphoric acids is used as a catalyst.

2. The process for producing 2-adamantanol and 2-adamantanone according to claim 1, wherein the said solid acid is at least one kind selected from zeolite, zirconia, silica-alumina, alumina, heteropoly acid, and cation exchange resin.

3. The process for producing 2-adamantanol and 2-adamantanone according to claim 1 or 2, wherein the amount of at least one kind selected from the group consisting of carboxylic acids, sulfonic acids, and phosphoric acids coexisting with the acid catalyst is 150 molar ratio or less against 1-adamantanol.

4. The process for producing 2-adamantanol and 2-adamantanone according to any one of claims 1 to 3, wherein the reaction temperature is in a range from 30 to 250°C.

5. The process for producing 2-adamantanol and 2-adamantanone according to any one of claims 1 to 4, wherein the acid coexisting with the acid catalyst is at least one kind selected from halogenated carboxylic acid, halogenated sulfonic acid, and alkyl sulfonic acid.
